(19) Europäisches Patentamt
European Patent Office
Office européen des brevets

(11) EP 2 376 056 B1

(12) FASCICULE DE BREVET EUROPEEN

(45) Date de publication et mention de la délivrance du brevet:
**07.06.2017 Bulletin 2017/23**

(21) Numéro de dépôt: **09795417.6**

(22) Date de dépôt: **17.12.2009**

(51) Int Cl.:
***A61K 8/362*** (2006.01) ***A61K 8/44*** (2006.01)
***A61K 31/085*** (2006.01) ***A61K 31/165*** (2006.01)
***A61K 31/194*** (2006.01) ***A61Q 19/00*** (2006.01)
***A61P 17/00*** (2006.01)

(86) Numéro de dépôt international:
**PCT/EP2009/067416**

(87) Numéro de publication internationale:
**WO 2010/070049 (24.06.2010 Gazette 2010/25)**

# (54) FORMULATIONS COSMETIQUES ET PHARMACEUTIQUES DE MOLECULES DE CALIXARENES

KOSMETISCHE UND PHARMAZEUTISCHE FORMULIERUNGEN VON CALIXAREN-MOLEKÜLEN

COSMETIC AND PHARMACEUTICAL FORMULATIONS OF CALIXARENE MOLECULES

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **17.12.2008 FR 0858703**

(43) Date de publication de la demande:
**19.10.2011 Bulletin 2011/42**

(73) Titulaire: **Institut de Radioprotection et de Sûreté Nucléaire**
**92260 Fontenay aux Roses (FR)**

(72) Inventeurs:
- **SPAGNUL, Aurélie**
  **06000 Nice (FR)**
- **REBIERE, François**
  **F-92160 Antony (FR)**
- **PHAN, Guillaume**
  **F-92120 Montrouge (FR)**
- **BOUVIER-CAPELY, Céline**
  **F-92320 Chatillon (FR)**
- **FATTAL, Elias**
  **F-75012 Paris (FR)**

(74) Mandataire: **Regimbeau**
**20, rue de Chazelles**
**75847 Paris Cedex 17 (FR)**

(56) Documents cités:

| | |
|---|---|
| **WO-A-02/44121** | **WO-A-95/19974** |
| **WO-A-99/24396** | **WO-A-99/55386** |
| **WO-A-03/024583** | **WO-A-2004/085442** |
| **WO-A-2005/123660** | **DE-A1- 10 352 466** |
| **FR-A- 2 782 007** | **FR-A- 2 899 814** |
| **FR-A- 2 904 782** | **US-A- 5 441 983** |
| **US-A- 5 489 612** | |

- **ARCHIMBAUD M ET AL: "Application of calixarenes for the decorporation of uranium: Present limitations and further trends" RADIATION PROTECTION DOSIMETRY, NUCLEAR TECHNOLOGY PUBLISHING, GB, vol. 53, no. 1-4, 1 janvier 1994 (1994-01-01), pages 217-220, XP009120911 ISSN: 0144-8420**
- **TYMEN H ET AL: "Contamination and decontamination of rat and human skin with plutonium and uranium, studied with a Franz's chamber" INTERNATIONAL JOURNAL OF RADIATION BIOLOGY, TAYLOR AND FRANCIS, LONDON, GB, vol. 76, no. 10, 1 octobre 2000 (2000-10-01), pages 1417-1424, XP009120931 ISSN: 0955-3002**
- **TRAN MINH B ET AL: "Percutaneous absorption of metallic cations: Particularities of uranium" ANNALES PHARMACEUTIQUES FRANCAISES, MASSON, PARIS, FR, vol. 57, no. 6, 1 novembre 1999 (1999-11-01), pages 462-467, XP009120930 ISSN: 0003-4509**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention concerne le domaine des traitements des contaminations cutanées par les actinides.

Les activités de l'industrie nucléaire nécessitent chaque année la manipulation de dizaines de milliers de tonnes d'uranium au cours des diverses étapes de traitement du combustible nucléaire : extraction des mines uranifères, purification chimique, enrichissement isotopique, fabrication des éléments combustibles, retraitement du combustible irradié, suivi de son entreposage et de son stockage. L'uranium ou les actinides transuraniens (plutonium ou américium par exemple) sont ainsi manipulés dans les installations impliquées dans le cycle du combustible, mais également au sein des laboratoires de recherche travaillant aussi bien sur l'amont que sur l'aval du cycle.

Afin d'assurer la protection des travailleurs du secteur nucléaire, l'exposition aux rayonnements ionisants associée à une pratique doit être la plus faible possible. Pour ce faire, les lieux d'exposition potentielle sont soumis à d'importantes contraintes techniques permettant ainsi d'assurer une surveillance portant aussi bien sur l'exposition à l'irradiation externe (balises d'alarme $\gamma$, dosimètre individuel) que sur la contamination interne (appareils de prélèvement d'aérosols, anthroporadiamétrie pulmonaire, dosage de l'uranium urinaire et fécal) des travailleurs potentiellement exposés [1]. Cependant, malgré les moyens de protection mis en oeuvre au poste de travail, un certain nombre d'accidents d'exposition du travailleur du nucléaire sont recensés. Les principales voies d'incorporation des actinides dans l'organisme sont l'inhalation, l'ingestion et la voie cutanée (contamination surfacique et/ou blessure).

La contamination par blessure reste très préoccupante puisqu'elle peut être à l'origine d'une contamination interne significative et durable des individus après transfert des actinides dans l'organisme. En effet, dans le cas d'une blessure accidentelle entraînant une contamination par les actinides tels que les composés solubles d'uranium, le transfert des actinides au travers d'une peau excoriée et donc la résorption par la circulation sanguine peuvent survenir très rapidement (30 minutes). De plus, si la décontamination d'une blessure superficielle n'est que partielle, l'uranium reste biologiquement disponible car l'épiderme viable se comporte comme un réservoir d'uranium [2]. Sont observés alors au niveau cutané des effets cytotoxiques dits précoces. L'uranium transite ensuite dans l'organisme par l'intermédiaire de la circulation systémique et une première partie de cet uranium est éliminée par excrétion urinaire. La quantité de radioélément demeurant dans l'organisme se dépose au niveau de deux principaux organes cibles que sont les reins et le squelette. Des effets dits différés peuvent se manifester alors au niveau de ces organes, témoignant ainsi de la toxicité chimique et radiologique à long terme de l'uranium notamment pour le cas des composés les plus actifs (uranium enrichi) [2-4].

Les méthodes actuelles de décontaminations cutanées consistent, après transfert des individus au service médical, à nettoyer la peau à l'aide d'une solution désinfectante adaptée additionnée d'agent chélateur, tel que l'acide Diéthylène Triamine Penta Acétique (DTPA) à 25 % par exemple, dans le cas des blessures par plaies. Le nettoyage des plaies peut ensuite être complété par un parage chirurgical des tissus contaminés en cas de contaminations par des particules. Dans le cas de composés solubles de l'uranium, il est important de souligner le fait que le délai entre le moment de la contamination et l'arrivée au service médical (quinze minutes en moyenne) est suffisant pour permettre une diffusion de l'uranium au travers de la couche cornée de l'épiderme, ce qui conduit à la formation d'un réservoir d'uranium qui pourra ensuite diffuser plus profondément [5],

Or, il n'existe aucune mesure d'urgence alternative permettant d'empêcher ou de limiter de manière satisfaisante le transfert des actinides à travers une plaie au moment et sur le lieu de l'accident. De plus, les agents chélateurs disponibles en clinique tel que le DTPA ne sont pas capables de complexer sélectivement les composés d'uranium et leur efficacité demeure très limitée en milieu biologique.

La thérapie de choix en cas de contamination interne par les actinides consiste ensuite à injecter à la victime par voie systémique des solutions contenant des agents décorporants qui accélèrent l'élimination urinaire des actinides incorporés (ex. solution de DTPA pour Pu et Am). La solution de DTPA n'étant pas efficace pour la décorporation de l'uranium, d'autres agents décorporants potentiellement efficaces tels que des ligands catécholates (CAM) et hydroxypyridinonates (HOPO) ont été proposés, mais ne sont pas encore cliniquement acceptés [6, 7]. Le ligand éthane-1-hydroxy-1,1-bisphosphonate (EHBP) se trouve être un aussi bon agent complexant de l'uranium que HOPO et CAM et présente l'avantage d'être déjà utilisé cliniquement dans d'autres indications [8]. Cependant, l'action décorporante de ces agents dépend fortement du temps écoulé entre le moment de la contamination et l'injection [8].

Au final, l'absence de mesure d'urgence à administrer au moment et sur le lieu de l'accident constitue une lacune qu'il conviendrait de combler afin de limiter la pénétration potentielle des actinides dans l'organisme à partir de la blessure ou de la surface de contamination cutanée. La recherche d'un agent de décontamination visant à réduire le flux transcutané des actinides suite à une contamination par contact ou par blessure est un sujet d'actualité sur lequel un nombre relativement restreint de travaux ont été menés [3, 4, 9-11].

La demande WO 2006/123051 décrit l'utilisation des p-tert-butylcalix[6]arènes fixés sur un support pour complexer sélectivement l'uranium, l'américium et le plutonium de l'urine.

La demande WO 95/19974 décrit l'utilisation des calix[3] à [8]arènes pour le traitement des infections bactériennes ou fongiques, du VIH et du cancer.

La demande US 5,441,983 décrit l'utilisation de calix[6]arènes pour le traitement d'infections virales et du cancer.

La demande FR 2 782 007 décrit l'utilisation de calix[6]arènes pour le traitement des maladies fibrotiques.

La demande WO 02/44121 décrit l'utilisation de calix[4]arènes pour le traitement d'infections virales.

Le document Archimbaud M et al. (Radiation Protection Dosimetry, Nuclear Technology Publishing, vol 53, no. 1-4; 1 janvier 1994) décrit l'utilisation des calixarènes pour la décorporation de l'uranium chez des rats par voie intrapéritonéale.

La demande WO 2004/085442 décrit l'utilisation des calixarènes pour la détoxification de patients suite à une intoxication causée par des métaux lourds ou des médicaments.

La demande WO 03/024583 décrit des compositions colloïdales dispersibles comprenant des nanoparticules de calixarènes amphiphiles véhiculant des agents actifs pour les libérer au contact de la peau.

Aucun de ces documents ne décrit l'utilisation des calixarènes pour une administration par voie topique pour le traitement des contaminations cutanées par des actinides.

Les calixarènes sont des cages complexantes particulières. En 1993, Araki et coll. ont montré les propriétés complexantes en extraction liquide-liquide, du 1,3,5-O-triméthyl-2,4,6-O-triacide carboxylique-para-tertio-butyl-[calix6]-arène (molécule représentée ci-après par la formule IA), vis-à-vis de l'uranium (Chem. Lett., 829-832, 1993). En 1994, Van Duynoven et coll. ont utilisé la même molécule pour étudier ses équilibres conformationnels (J. Am. Chem. Soc., 116, 5814-5822). En 1997, C. Dinse et coll. (Radioprotection, 32 n° 5, 659-671) ont démontré la sélectivité, en extraction liquide-liquide de la molécule de formule (IA) ci-dessous vis-à-vis de l'uranium en présence de plutonium et de sodium.

Aucun des produits ou techniques de l'art antérieur ne permet de proposer une mesure topique d'urgence afin de limiter voire supprimer la pénétration dans l'organisme des actinides à partir d'une contamination cutanée.

De façon surprenante, les inventeurs ont montré qu'une nanoémulsion huile-dans-eau comprenant des p-tert-butylcalix[6]arènes dans la phase huileuse permet de fixer l'uranium et empêche sa diffusion transdermique.

La présente invention a ainsi pour objet une composition dermatologique ou dermatocosmétique selon l'objet de la revendication 1 comportant un calixarène, de préférence un calix[6]arène pour son utilisation dans le traitement des contaminations cutanées par des actinides.

Avantageusement, le calixarène selon l'invention est un para-tertio-butyl calix[6]arène de formule (IA) ou (IB) :

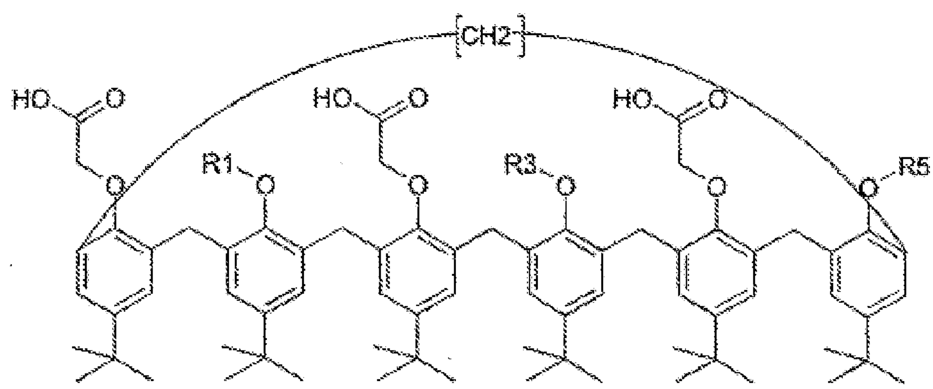

(IA)

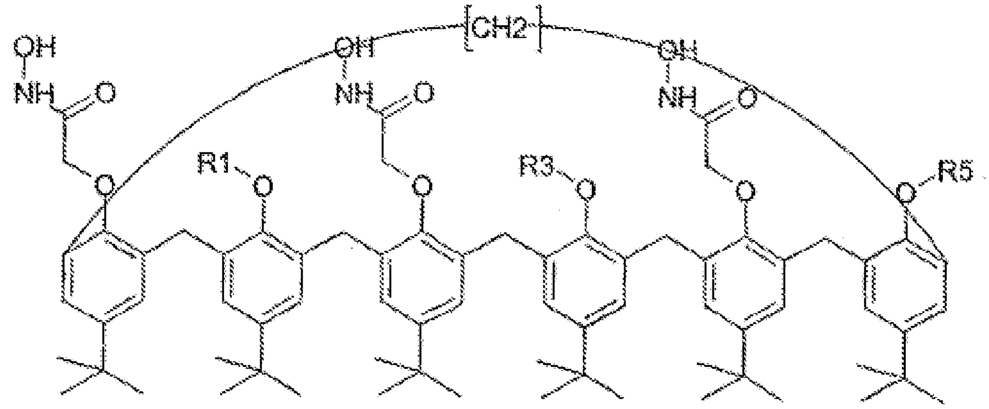

(IB)

où R1, R3 et R5, identiques ou différents, représentent, chacun indépendamment :

(i) un atome d'hydrogène, ou d'halogène,

(ii) un radical acétyle, amino, phosphate, nitro, sulfate, carboxy, carboxylique, thiocarboxy, carbamate, thiocarbamate,

(iii) un alkyle linéaire ou ramifié, ayant 1 à 60, de préférence 1 à 30 atomes de carbone, éventuellement substitué, présentant éventuellement au moins une insaturation éthylénique ou acétylénique,

(iv) un cycloalkyle ayant de 3 à 12 atomes de carbone, éventuellement substitué, présentant éventuellement au moins une insaturation éthylénique ou acétylénique,

(v) un aryle, un naphtyle, un aryl-(alkyle en C1-C30), un(alkyl en C1-C30)-aryle, éventuellement substitués,

les radicaux (ii) à (v) pouvant être substitués par des atomes d'halogène, des organo-métalliques, des fonctions alcools, aminées, acides ou esters carboxyliques, sulfoniques, sulfuriques, phosphoriques, phosphoniques ou hydroxamiques, carbamates, thiocarbamates, éthers, thiols, époxydes, thioépoxydes, isocyanates, isothiocyanates ou un carbone de ces radicaux pouvant être remplacé par un hétéroatome d'azote, soufre, phosphore, oxygène, bore, arsenic ;

(vi) un polysaccharide.

Selon un mode de réalisation avantageux, dans la formule (IA) ou (IB), deux parmi R1, R3 et R5 représentent hydrogène ou méthyle, le troisième étant un polysaccharide.

Avantageusement, lesdits polysaccharides sont choisis dans le groupe constitué des dextranes, du chitosane et des agaroses.

Selon un autre mode de réalisation avantageux, les para-tertio-butyl calix[6]arènes de l'invention sont des composés de formule (IA) ou (IB) dans laquelle R1, R3 et R5 sont identiques et de préférence ils représentent un hydrogène.

Un autre objet de la présente invention est l'utilisation d'un calixarène pour la préparation d'un médicament destiné au traitement des contaminations cutanées par des actinides.

Un autre objet de la présente invention est une méthode de traitement des contaminations cutanées par des actinides comprenant l'application sur la peau d'une quantité efficace de calixarène.

La série chimique des actinides comprend les éléments chimiques du tableau périodique se situant entre l'actinium et le lawrencium, possédant donc un numéro atomique entre 89 et 103 inclus, soit l'actinium (Ac), le Thorium (Th), le Protactinium (Pa), Uranium U, Neptunium Np, Plutonium Pu, Americium Am, Curium Cm, Berkelium Bk, Californium Cf, Einsteinium Es, Fermium Fm, Mendelevium Md, Nobelium No, Lawrencium (Lw).

Les actinides sont tous des éléments radioactifs et tirent leur nom de l'actinium (Z=89), un métal lourd, car ils possèdent des propriétés chimiques voisines.

L'actinide produit le plus abondamment dans le cycle du combustible nucléaire est le plutonium, avec en tête son principal isotope le plutonium-239, lui-même fissile. Mais les réacteurs nucléaires génèrent, en quantité moindre, d'autres actinides qui sont appelés « mineurs » pour cette raison. Les principaux sont le neptunium-237, les américium-241 et 243 et les curium-244 et 245. Les actinides mineurs constituent avec les produits de fission une grande partie des déchets radioactifs à haute activité et longue durée de vie (HALV), c'est-à-dire les déchets de l'industrie électronucléaire les plus dangereux.

Avantageusement, l'invention est utilisée pour le traitement des contaminations cutanées par l'uranium, le plutonium et/ou l'américium.

Un autre objet de la présente invention est l'utilisation des calixarènes, avantageusement des calix[6]arènes, pour empêcher ou limiter le transfert d'un actinide dans l'organisme à partir d'un site de contamination cutanée, avantageusement pour empêcher ou limiter le transfert d'uranium, plutonium et/ou américium dans l'organisme à partir d'un site de contamination cutanée.

L'invention permet en effet de proposer une composition dermocosmétique qui va permettre d'empêcher la pénétration des actinides dans l'organisme à partir d'un site de contamination cutanée ou de limiter la quantité d'actinide pénétrant dans l'organisme à une quantité insuffisante pour déclencher un symptôme lié à la contamination, en particulier un symptôme rénal ou osseux.

Un autre objet de la présente invention est une composition dermocosmétique comprenant un ou plusieurs composés calixarène à titre de principe actif et un ou plusieurs excipients cosmétiquement acceptables.

« Composition dermocosmétique » signifie composition cosmétique destinée à une application sur la peau.

Un autre objet de la présente invention est une composition dermatologique comprenant un ou plusieurs composés calixarène à titre de principe actif et un ou plusieurs excipients pharmaceutiquement acceptables.

« Composition dermatologique » signifie composition pharmaceutique destinée à une application sur la peau.

Avantageusement, les excipients de la formulation selon l'invention sont choisis conformes à la Pharmacopée Française.

Avantageusement, les excipients de la formulation dermocosmétique ou dermatologique selon l'invention ne constituent pas des promoteurs de pénétration cutanée qui pourraient désorganiser la structure cutanée et promouvoir

la pénétration cutanée des actinides contaminants.

Etant donné le fort caractère lipophile des molécules de calixarène, la composition dermocosmétique ou dermatologique selon l'invention se présente avantageusement sous une forme comprenant une phase huileuse, le ou les composés calixarènes se trouvant dans la phase huileuse, comme :

- un oléogel dont la base est une huile ;
- une pâte anhydre dont la base est une phase grasse solide ou une cire ;
- une émulsion huile dans eau (H/E) constituée par la dispersion de gouttelettes d'huile dans de l'eau ou eau dans huile (E/H) constituée par la dispersion de gouttelettes d'eau dans la phase huileuse.

Avantageusement, la composition dermocosmétique ou dermatologique selon l'invention est une nanoémulsion huile-dans-eau ou eau-dans-huile, de préférence une nanoémulsion huile-dans-eau.

On appelle une « nanoémulsion » une émulsion dont la taille des gouttelettes est comprise entre 50 et 200 nm.

L'émulsion, et en particulier la nanoémulsion (diamètre moyen des gouttelettes dispersées inférieur au micromètre), s'est avérée être une formulation particulièrement avantageuse car elle est peu rémanente et donc facilement lavable permettant ainsi d'envisager une décontamination plus efficace. De plus, elle est susceptible d'assurer une meilleure disponibilité des calixarènes à la surface de la peau et présente une grande surface totale développée grâce aux gouttelettes d'huile finement dispersées dans la phase aqueuse.

Avantageusement, une composition dermocosmétique ou dermatologique selon l'invention sous forme de nanoémulsion présente des gouttelettes de taille comprise entre 50 et 200 nm, préférentiellement entre 100 et 200 nm.

Avantageusement, la phase huileuse est composée d'huiles minérales, végétales, de synthèse ou un de leurs mélanges. Préférentiellement, la phase huileuse comprend de la paraffine. De manière particulièrement préférée, la phase huileuse est constituée de paraffine.

Avantageusement, au moins un des composés calix[6]arène présents dans la composition dermatologique selon l'invention à titre de principe actif présente la formule (IA) ou (IB) telle que définie précédemment où R1, R3 et R5 ont les définitions données précédemment.

Préférentiellement, tous les composés calix[6]arène présents dans la composition dermatologique selon l'invention à titre de principe actif présentent la formule (IA) ou (IB) telle que définie précédemment où R1, R3 et R5 ont les définitions données précédemment.

Préférentiellement, la composition dermatologique selon l'invention comprend 50 à 95%, préférentiellement 70 à 80% d'eau. Préférentiellement, la composition dermatologique selon l'invention comprend 5 à 50%, préférentiellement 15 à 25% de phase huileuse. Préférentiellement la composition dermatologique selon l'invention comprend de 1 à 15% de tensio-actifs. Avantageusement, la composition dermatologique selon l'invention comprend 2 à 30 mg, de préférence, 2 à 12 mg de calix[6]arène par gramme de la composition. Lorsque la composition dermatologique selon l'invention est une nanoémulsion, elle comprend 2 à 30 mg, de préférence 2 à 12 mg de calix[6]arène par gramme de la composition.

Avantageusement, au moins un des composés calixarène présents dans la composition dermocosmétique selon l'invention est un calix[6]arène.

Préférentiellement, tous les composés calixarène présents dans la composition dermocosmétique selon l'invention à titre de principe actif sont des calix[6]arène.

Avantageusement, au moins un des composés calixarène présents dans la composition dermocosmétique selon l'invention à titre de principe actif présente la formule (IA) ou (IB) telle que définie précédemment où R1, R3 et R5 ont les définitions données précédemment.

Préférentiellement, tous les composés calixarène présents dans la composition dermocosmétique selon l'invention à titre de principe actif présentent la formule (IA) ou (IB) telle que définie précédemment où R1, R3 et R5 ont les définitions données précédemment.

Préférentiellement, la composition dermocosmétique selon l'invention comprend 50 à 95%, préférentiellement 70 à 80% d'eau. Préférentiellement, la composition dermocosmétique selon l'invention comprend 5 à 50%, préférentiellement 15 à 25% de phase huileuse. Préférentiellement la composition dermocosmétique selon l'invention comprend de 1 à 15% de tensio-actifs. Avantageusement, la composition dermocosmétique selon l'invention comprend 2 à 30 mg, de préférence, 2 à 12 mg de calixarène par gramme de la composition. Lorsque la composition dermocosmétique selon l'invention est une nanoémulsion, elle comprend 2 à 30 mg, de préférence 2 à 12 mg de calixarène par gramme de la composition.

## Figures

Figure 1 : Schéma d'une émulsion huile-dans-eau,
Figure 2 : Mode de réalisation des nanoémulsions,
Figure 3 : Evolution de la taille des gouttelettes huileuses dispersées en fonction de la concentration en calixarène dans les nanoémulsions,
Figure 4 : Evolution du potentiel zêta des gouttelettes huileuses en fonction de la concentration en calixarène dans l'émulsion (A). Hypothèse de localisation de molécules de calixarène à la surface des gouttelettes d'huile de l'émulsion (B),
Figure 5: Evolution du pH des émulsions en fonction de la concentration en calixarène incorporée (A). Hypothèse de localisation de molécules de calixarène à la surface des gouttelettes d'huile de l'émulsion (B),
Figure 6 : Pourcentage d'uranium retenu par le filtre du système d'ultrafiltration en fonction de la concentration en uranium déposé,
Figure 7 : Répartition de l'uranium entre le rétentat, le filtre et le filtrat après ultrafiltration (A). Représentation du système d'ultrafiltration (B),
Figure 8 : Pourcentage d'uranium extrait en fonction du temps de contact entre la solution contaminante et la nanoémulsion ainsi qu'en fonction du type de contact (dynamique et statique). *Remarque : temps de contact représenté = durée réelle de l'expérimentation + 30 minutes d'ultrafiltration*
Figure 9: Pourcentage d'uranium extrait en fonction du temps de contact entre la solution contaminante et la dispersion de calixarène dans de l'huile de paraffine ainsi qu'en fonction du type de contact (dynamique et statique). *Remarque : temps de contact représenté = durée réelle de l'expérimentation + 20 minutes de traitement*
Figure 10 : Schémas des cellules de Franz utilisées pour l'étude du passage transcutané de l'uranium ou des molécules de calixarène à partir des formulations.
Figure 11 : Cinétique de diffusion transcutanée de l'uranium sur 24 heures en cellule de Franz (n = 5), en l'absence ou en présence de nanoémulsion de calixarène.

Les exemples suivants illustrent l'invention sans en limiter la portée.

## Exemples

### Exemple 1 : Préparation d'une nanoémulsion huile-dans-eau de calix[6]arène

On prépare une nanoémulsion huile-dans-eau; dans lesquelles les molécules de calixarènes sont incorporées dans la phase huileuse. Ces nanoémulsions sont constituées d'une huile minérale (huile de paraffine), de tensioactifs non ioniques (Tween 80® et Span 80®), d'eau MilliQ et de 1,3,5-OMe-2,4,6-$OCH_2COOH$-p-ter-butylcalix[6]arène. Les proportions massiques de tensio-actifs, d'huile et d'eau ont été respectivement fixées à 5 %, 20 % et 75 %. La proportion massique relative des deux tensioactifs a été optimisée de façon à obtenir les conditions optimales d'émulsification (balance hydrophile-lipophile HLB=11).

Les nanoémulsions de calixarène ont été préparées selon la méthode d'inversion de phase. La phase huileuse de l'émulsion correspondant au mélange huile-calixarène-tensioactifs et la phase aqueuse sont dans un premier temps placées séparément dans un bain thermostaté à 50°C sous agitation douce durant trente minutes. Une fois ce délai d'équilibration en température écoulé, la phase aqueuse est ajoutée au goutte-à-goutte à la phase huileuse sous agitation douce (figure 2). Les nanoémulsions ainsi obtenues sont conservées sous agitation dans le bain thermostaté durant 45 minutes puis sont placées sous agitation à température ambiante durant 45 minutes. Les nanoémulsions sont ensuite conservées à température ambiante à l'abri de la lumière.

### Exemple 2: Caractérisation des nanoémulsions obtenues à l'Exemple 1

Les nanoémulsions ont été caractérisées par mesure de la taille des gouttelettes huileuses dispersées, du potentiel zêta (charge globale de surface que les gouttelettes acquièrent dans le milieu aqueux) et du pH. Le sens des émulsions a également été vérifié par conductimétrie et par le test de dilution de l'émulsion dans l'eau.

La taille moyenne des gouttelettes huileuses dispersées ainsi que l'indice de polydispersité (PDI) reflétant l'homogénéité de la distribution de taille ont été mesurés par spectroscopie de corrélation de photons.

Cette étude a montré que le diamètre des globules huileux est inférieur à 200 nm et qu'il diminue de façon significative ($p < 0,05$) lorsque la quantité de calixarène introduite est supérieure à 2 milligrammes par gramme d'émulsion (figure 3). Cette diminution de taille est corrélée à une augmentation significative ($p < 0,05$) de l'indice de polydispersité, traduisant ainsi une diminution de l'homogénéité des émulsions. Cependant, la valeur du PDI demeure faible (< 0,250), ce qui indique que la distribution de taille des gouttelettes huileuses reste homogène pour des nanoémulsions chargées en calixarène jusqu'à 8 mg/g.

Le potentiel zêta a été déterminé par mesure de la mobilité électrophorétique des globules huileux dispersés,

Cette mesure a permis de mettre en évidence la présence de calixarène à la surface de ces gouttelettes (figure 4). En effet, l'augmentation de la concentration en calixarène dans les nanoémulsions entraîne une diminution de la charge de surface des gouttelettes huileuses jusqu'à la concentration de 4 mg/g, concentration au delà de laquelle le potentiel zêta tend à se stabiliser. Cette stabilisation peut être expliquée par une probable déprotonation de molécules de calixarène qui se trouveraient en surface des globules huileux.

La présence de calixarène à la surface des gouttelettes huileuses dispersées a également été mise en évidence par mesure pH-métrique. Une comparaison des pH d'émulsions non chargées en calixarène et d'émulsions chargées à 2, 4, 6 et 8 mg/g a montré une diminution de pH d'environ une unité en présence de l'agent chélatant. Ce phénomène s'expliquerait par une déprotonation de molécules de calixarène présentes en surface des globules huileux (figure 5).

Exemple 3 : Efficacité *in vitro* des nanoémulsions de calixarène pour la décontamination

L'efficacité des nanoémulsions de calixarène a été évaluée *in vitro* par mise en oeuvre de la technique d'ultrafiltration. En effet, l'ultrafiltration permet de récupérer une partie de la phase aqueuse des nanoémulsions dans laquelle il est alors possible de doser l'uranium libre par spectrométrie de masse couplée à un plasma inductif (ICP-MS).

Dans un premier temps, les paramètres d'ultrafiltration-ultracentrifugation ont été optimisés de façon à n'obtenir dans le filtrat que la phase aqueuse des nanoémulsions :

- porosité du filtre : 3000 kDa
- volume à centrifuger : 400 $\mu$l
- température de centrifugation : 20 °C
- vitesse de centrifugation : 6000 tours par minute
- durée de centrifugation : 30 min

Les filtrats limpides ainsi obtenus ont été analysés par chromatographie liquide haute performance (HPLC). L'analyse HPLC a montré que les filtrats sont uniquement constitués d'eau indiquant ainsi l'absence d'huile, de tensioactifs et de calixarène.

Une étude visant à évaluer la rétention de l'uranium par les filtres utilisés en ultrafiltration a alors été menée. Cette étude a permis de conclure que 47 % en moyenne de l'uranium déposé sur le filtre sous forme de nitrate d'uranyle en tampon acétate pH = 5, est retenu dans ce dernier lors de l'ultrafiltration (figure 6). Ceci est dû au fait que le filtre est constitué de cellulose régénérée, matériau qui présente des sites chargés électro négativement avec lesquels l'uranium peut interagir par liaisons électrostatiques et ainsi être retenu [16]. Il a également été vérifié par la réalisation d'un bilan de matière que ce pourcentage correspondait bien à de l'uranium retenu dans le filtre et non à un phénomène de concentration de l'uranium dans la phase déposée sur le filtre (rétentat) par la réalisation d'un bilan de matière (figure 7). En effet, il n'y a pas d'augmentation de la concentration en uranium dans le rétentat après ultrafiltration par rapport à la concentration initialement déposée.

Sachant qu'en utilisant la technique d'ultrafiltration il est possible de récupérer des filtrats de nanoémulsion constitués uniquement d'eau et que l'uranium est retenu par le filtre des systèmes d'ultrafiltration à hauteur de 47 % en moyenne, il est donc possible d'évaluer *in vitro* le pouvoir de décontamination des nanoémulsions de calixarène.

Ce test d'efficacité de décontamination des nanoémulsions de calixarène a été mené selon deux modes de contact entre le contaminant et la nanoémulsion :

◦ *contact dynamique :* agitation à retournement pendant une durée déterminée d'un tube contenant la solution contaminante et la nanoémulsion de calixarène.

◦ *contact statique :* mise en contact pendant une durée déterminée de la nanoémulsion de calixarène avec la solution contaminante sans agitation.

Une cinétique d'extraction de l'uranium par les nanoemulsions de calixarène a ainsi pu être réalisée (pour un rapport $\frac{Qcalixarène}{Quranium} = 10000$ et $\frac{Vcontamination}{Vformulation} = 1$ (figure 8)).

Cette étude montre que les nanoémulsions non chargées en calixarène extraient environ 25% de l'uranium en mode statique et 40% en mode dynamique. Ceci peut être expliqué par un piégeage de l'uranium par les tensioactifs non ioniques, car ces derniers présentant des groupements éthoxyle susceptibles d'établir des liaisons hydrogènes avec les ions hydroxyles [17] et donc également aptes à retenir de l'uranium. Le fait d'agiter le mélange nanoémulsion non chargée en calixarène et solution contaminante permet de favoriser le contact entre l'uranium et les tensioactifs, ce qui peut expliquer que l'extraction de l'uranium soit plus importante dans les conditions dynamiques.

Concernant les nanoémulsions chargées en calixarène à 2 mg/g, environ 90% de l'uranium est extrait dès 35 minutes (soit 5 minutes en temps réel d'expérimentation) en dynamique contre environ 85 % en mode statique. Ces résultats montrent donc que la présence de calixarène dans les nanoémulsions améliore d'au moins un facteur 2 le rendement d'extraction de l'uranium.

De plus, une étude cinétique précédemment réalisée avec de l'huile de paraffine chargée en calixarène en tant que formulation galénique, a montré que moins de 10 % de l'uranium était extrait par cette formulation en mode statique, toutes conditions restant égales par ailleurs $\frac{Qcalixarène}{Quranium} = 10000$ et $\frac{Vcontamination}{Vformulation} = 1$ (figure 9).

Il apparaît donc clairement que la formulation choisie (nanoémulsion de calixarène) est beaucoup plus efficace que l'excipient (huile de paraffine) lui-même chargé en calixarène. Ceci est très probablement dû au fait que la surface développée entre le calixarène et l'uranium dans les nanoémulsions est beaucoup plus grande.

En conclusion, ces résultats montrent que la formulation choisie (nanoémulsion) et le principe actif utilisé, c'est-à-dire le calixarène, contribuent à l'efficacité de la forme galénique. Les nanoémulsions de calixarène sont donc efficaces en décontaminatton statique. Ce sont donc des systèmes tout à fait adaptés à la décontamination des plaies.

<u>Exemple 4 : Efficacité *ex vivo* des nanoémulsions de calixarènes pour la décontamination</u>

L'efficacité des nanoémulsions de calixarène a été évaluée en utilisant un système de cellule de Franz par l'étude de la cinétique de passage transcutané de l'uranium avec et sans application de nanoémulsion (figure 10). Toutes les études de diffusion transdermique ont été menées sur des explants de peau d'oreille de porc en condition occlusive (n = 5). La contamination a été réalisée par dépôt d'une solution d'uranium appauvri ($^{238}U$) en tampon acétate pH = 5.

Une première expérimentation visant à déterminer la cinétique du passage transcutané du calixarène relargué depuis 1 ml de nanoémulsion chargée à 4 mg/g a montré que le calixarène ainsi formulé ne passait pas au travers de la peau sur une période d'exposition de 24 heures (détection en HPLC à une longueur d'onde $\lambda$ = 210 nm : limite de détection LD = 17 $ng.ml^{-1}$ ; limite de quantification LQ= 52 $ng.ml^{-1}$).

L'étude d'efficacité de décontamination d'une nanoémulsion chargée en calixarène carboxylique à 4 mg/g (rapport $Q_{calixarène}/Q_{uranium}$ = 10 000) a permis de mettre en évidence une diminution de 98% de la quantité d'uranium ayant diffusé à travers la peau au bout de 24 heures.

En effet, le traitement de la contamination par dépôt d'une nanoémulsion non chargée en calixarène permet déjà une diminution de 71% de la quantité d'uranium diffusé. Ceci est dû d'une part à la dilution de l'uranium dans la phase aqueuse de l'émulsion et d'autre part à un piégeage d'une partie de l'uranium par les constituants de la nanoémulsion (démontré dans les tests *in vitro)*. L'ajout de calixarène à la nanoémulsion permet encore de diminuer de 93% la quantité d'uranium diffusé par rapport à la nanoémulsion non chargée (figure 11)

En conclusion, ces résultats confirment l'efficacité des nanoémulsions de calixarène pour la décontamination des peaux contaminées par de l'uranium, puisqu'elles permettent de réduire le transfert transcutané de cet actinide.

Références bibliographiques

[1] Le Guen B and Bérard P. Uranium et ses composés. In: Toxicologie-Pathologie professionnelle, Paris; Elsevier, 1998, Encyclopédie Médico Chirurgicale 16-008-10.

[2] Petitot F, Moreels AM and Paquet F. In vitro évaluation of percutaneous diffusion of uranyl nitrate through intact or excoriated skin of rat and pig. Can J Physiol Pharmacol 82(2):133-9 (2004).

[3] Tymen H, Gerasimo P and Hoffschir D. Contamination and decontamination of rat and human skin with plutonium and uranium, studied with a Franz's chamber. Int J Radiat Biol 76(10):1417-24 (2000).

[4] Tymen H, Rateau G, Guillet K, Ramounet-Le Gall B, Gerasimo P and Fritsch P, Methods to measure transfer of radionuclides through intact or injured skin -Application to radiotoxicotogy [Méthodes de mesure du transfert cutané des actinides au travers d'un épiderme intact ou lésé, application à la radiotoxicologie]. Canadian Journal of Physiology and Pharmacology 80(7);733-741 (2002).

[5] De Rey BM, Lanfranchi HE and Cabrini RL. Percutaneous absorption of uranium compounds. Environmental Research 30(2):480-491 (1983).

[6] Durbin PW, Kullgren B, Xu J and Raymond KN. Development of Decorporatiàn Agents for the Actinides. Radiation Protection Dosimetry 79(1-4):433-443 (1998).

[7] Fukuda S, Lida H, Ikeda M, Yan X and Xie Y. Toxicity of uranium and the removal effects of CBMIDA and EHBP in simulated wounds of rats. Health Phys 89(1):81-8 (2005).

[8] Henge-Napoli MH, Ansoborlo E, Chazel V, Houpert P, Paquet F and Gourmeldn P. Effiacy of ethane-1-hydroxy-1,1-bisphosphonate (EHBP) for the decorporation of uranium after intramuscular contamination in rats. Int J Radiat Biol 75(11); 1473-7 (1999).
[9] Houpert P, Chazel V, Paquet F, Bailly T, Burgada R and Henge-Napoli MH Réduction of uranium transfer by local chelation iri simulated wounds in rats. Hum Exp Toxicol 20(5);237-41 (2001).
[10] Houpert P, Chazel V and Paquet F, A local approach to reduce industrial uranium wound contamination in rats. Can J Physiol Pharmacol 82(2):73-8 (2004).
[11] Yang Z, Xu K, Wang L, Gu H, Wei H, Xang M and Xu B. Self-assembly of small molecules affords multifonctional supramolecular hydrogels for topically treating simulated uranium wounds. Chem Commun 35:4414-4416 (2005).
[12] Shan Z, Yang Y-Z, Tao Z, Jian H and Chang-Hong L. Uranium(VI) extraction by Winsor microemulsion Systems using trialkyl phosphine oxide. Journal of Radioanalytical and Nuclear Chemistry 265(3):419-421 (2005).
[13] Guo J-x, Sun X, Du D-I, Wu X, Li M-x, Pang H, Sun S-x and Wang A-hi Uranium(VI) extraction from chloride solution with benzyloctadecyldimethyl ammonium chloride (BODMAC) in a liquid membrane process. Journal of Radioanalytical and Nuclear Chemistry 275(2):365-369 (2008).
[14] Seiller M and Martini MC. Formes pharmaceutiques pour application locale. Lavoisier, 1996,
[15] Martini M-C, Introduction à la dermopharrnacie et à la cosmétologie. Lavoisier 2006, p. 411.
[16] Guo L, Warnken KW and Santschi PH. Rétention behavior of dissolved uranium during ultrafiltration: Implications for colloïdal U in surface waters. Marine Chemistry 107(2):156-166 (2007).
[17] Liu W, Sun D, Li C, Liu Q and Xu J. Formation and stability of paraffin oil-in-water nano-emulsions prepared by the emulsion inversion point metbod. Journal of Colloid and Interface Science 303(2). : 557-563 (2006).

## Revendications

1. Composition dermatologique ou dermocosmétique sous la forme d'une nanoémulsion huile-dans-eau ou eau-dans-huile comprenant un ou plusieurs composés calixarène à titre de principe actif et un ou plusieurs excipients pharmaceutiquement respectivement cosmétiquement acceptables.

2. Composition dermatologique ou dermocosmétique selon la revendication 1, dans laquelle au moins un des composés calixarène est un calix[6]arène.

3. Composition dermatologique ou dermocosmétique selon la revendication 1 ou 2, dans laquelle au moins un des composés calixarène présente la formule (IA) ou (IB)

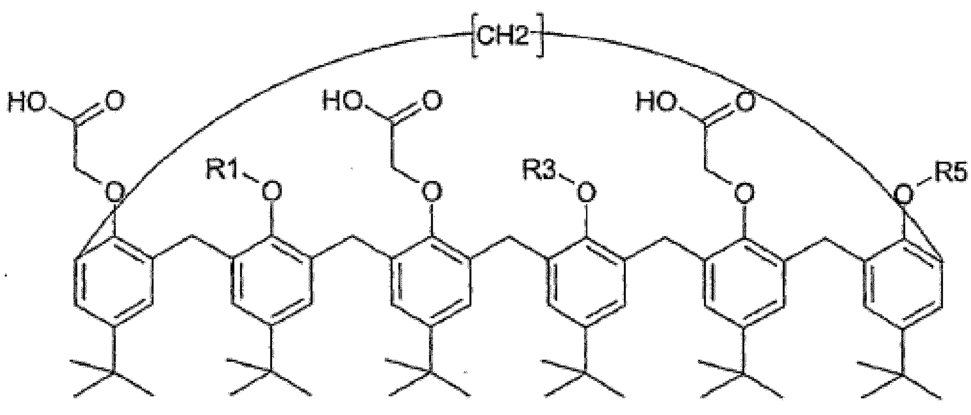

(IA)

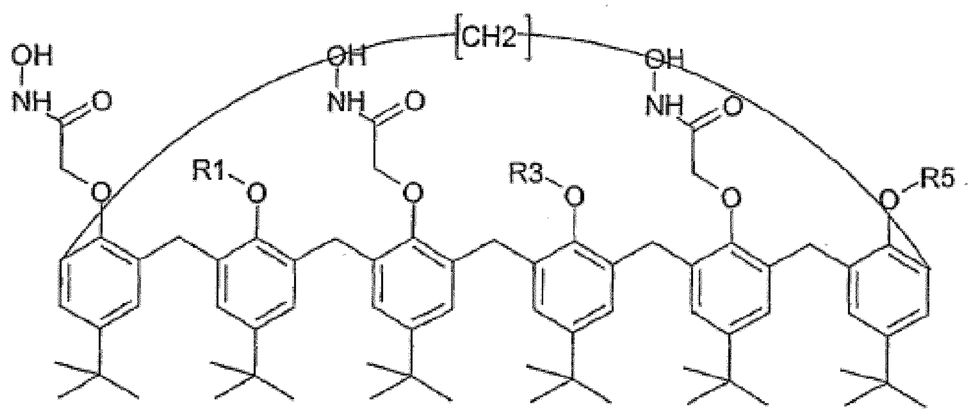

(IB)

où R1, R3 et R5, identiques ou différents, représentent, chacun indépendamment :

(i) un atome d'hydrogène, ou d'halogène,
(ii) un radical acétyle, amino, phosphate, nitro, sulfaten carboxy, carboxylique, thiocarboxy, carbamate, thiocarbamate,
(iii) un alkyle linéaire ou ramifié, ayant 1 à 60, de préférence 1 à 30 atomes de carbone, éventuellement substitué, présentant éventuellement au moins une insaturation éthylénique ou acétylénique,
(iv) un cycloalkyle ayant de 3 à 12 atomes de carbone, éventuellement substitué, présentant éventuellement au moins une insaturation éthylénique ou acétylénique,
(v) un aryle, un naphtyle, un aryl-(alkyl en C1-C30), un(alkyl en C1-C30)-aryle, éventuellement substitués, les radicaux (ii) à (v) pouvant être substitués par des atomes d'halogène, des organo-métalliques, des fonctions alcools, aminées, acides ou esters carboxyliques, sulfoniques, sulfuriques, phosphoriques, phosphoniques ou hydroxamiques, carbamates, thiocarbamates, éthers, thiols, époxydes, thioépoxydes, isocyanates, isothiocyanates ou un carbone de ces radicaux pouvant être remplacé par un hétéroatome d'azote, soufre, phosphore, oxygène, bore, arsenic,
(vi) un polysaccharide.

**4.** Composition dermocosmétique selon l'une quelconque des revendications 1 à 3, comprenant de 2 à 30 mg de calixarène par gramme de la composition.

## Patentansprüche

**1.** Dermatologische oder dermokosmetische Zusammensetzung in Form einer Öl-in-Wasser- oder Wasserin-Öl-Nanoemulsion, umfassend eine oder mehrere Calixaren-Verbindungen als Wirkstoff und einen oder mehrere pharmazeutisch beziehungsweise kosmetisch akzeptable Hilfsstoffe.

**2.** Dermatologische oder dermokosmetische Zusammensetzung nach Anspruch 1, wobei mindestens eine der Calixaren-Verbindungen ein Calix[6]aren ist.

**3.** Dermatologische oder dermokosmetische Zusammensetzung nach Anspruch 1 oder 2, wobei mindestens eine der Calixaren-Verbindungen die Formel (IA) oder (IB) aufweist:

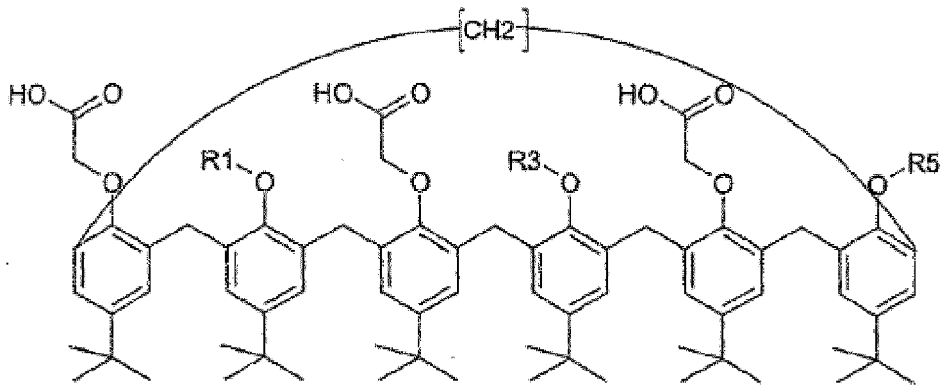

(IA)

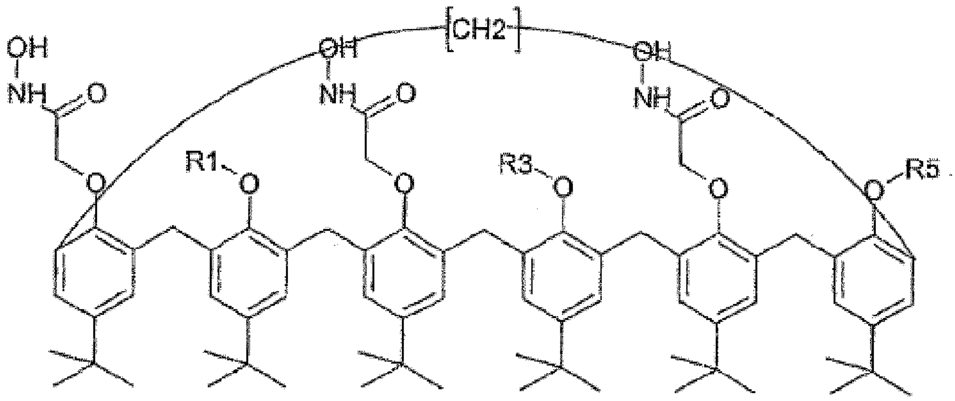

(IB)

wobei R1, R3 und R5, identisch oder unterschiedlich, jeweils unabhängig darstellen:

(i) ein Wasserstoff- oder Halogenatom,
(ii) ein Acetyl-, Amino-, Phosphat-, Nitro-, Sulfat-, Carboxy-, Carboxyl-, Thiocarboxy-, Carbamat-, Thiocarbamatradikal,
(iii) ein lineares oder verzweigtes Alkyl mit 1 bis 60, vorzugsweise 1 bis 30, Kohlenstoffatomen, eventuell substituiert, aufweisend eventuell mindestens einen ungesättigten Ethylen- oder Acetylenzustand,
(iv) ein Cycloalkyl mit 3 bis 12 Kohlenstoffatomen, eventuell substituiert, aufweisend eventuell mindestens einen ungesättigten Ethylen- oder Acetylenzustand,
(v) ein Aryl, ein Naphtyl, ein Aryl-(C1-C30-alkyl), ein (C1-C30-Alkyl)-aryl, eventuell substituiert,
wobei die Radikale (ii) bis (v) von Halogen-, Organometallatomen, Alkohol-, Amin-, Carboxyl-, Sulfon-, Sulfid-, Phosphor-, Phosphon- oder Hydroxam-, Carbamat-, Thiocarbamat-, Ether-, Thiol-, Epoxyd-, Thioepoxyd-, Isocyanat-, Isothiocyanatsäure- oder esterfunktionen oder einem Carbon dieser Radikale, möglicherweise von einem Stickstoff-, Schwefel-, Phosphor-, Sauerstoff-, Bor-, Arsenheteroatom ersetzt, substituiert sein können,
(vi) ein Polysaccharid.

**4.** Dermoskometische Zusammensetzung nach einem der Ansprüche 1 bis 3, umfassend 2 bis 30 mg Calixaren je Gramm der Zusammensetzung.

## Claims

**1.** A dermatological or dermocosmetic composition in the form of an oil-in-water or water-in-oil nanoemulsion comprising one or more calixarene compounds as an active ingredient and one or more pharmaceutically respectively cosmetically acceptable excipients.

**2.** The dermatological or dermocosmetic composition of claim 1, wherein at least one of the calixarene compounds is a calix[6]arene.

**3.** The dermatological or dermocosmetic composition of claim 1 or 2, wherein at least one of the calixarene compounds is of the formula (IA) or (IB)

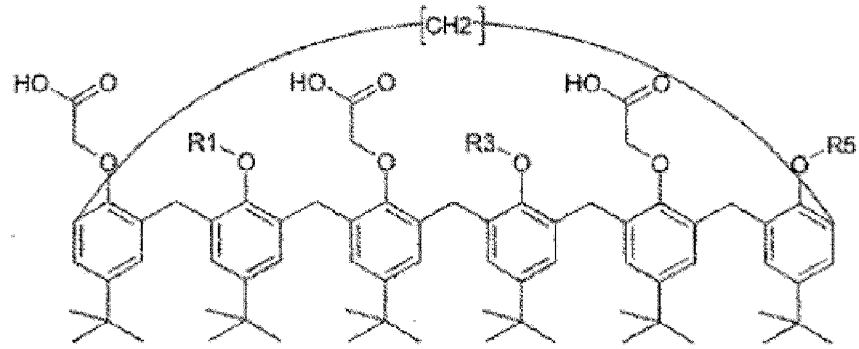

(IA)

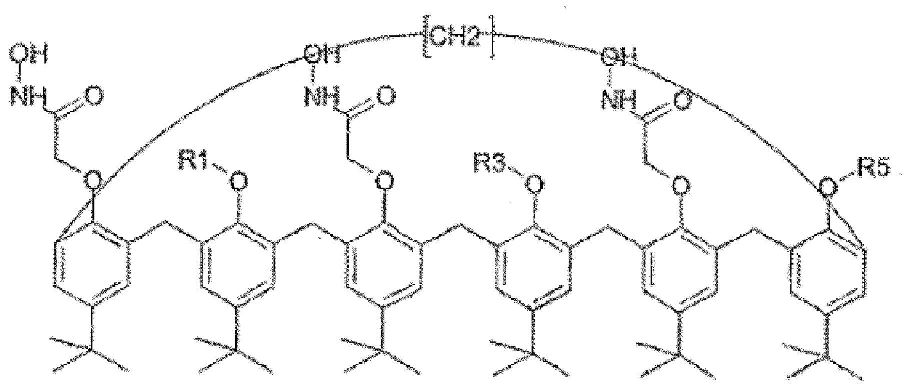

(IB)

wherein R1, R3 and R5, identical or different, each independently represent:

(i) a hydrogen or halogen atom,
(ii) an acetyl, amino, phosphate, nitro, sulfate, carboxy, carboxylic, thiocarboxy, carbamate or thiocarbamate radical,
(iii) an optionally substituted linear or branched alkyl having 1 to 60, preferably 1 to 30, carbon atoms which optionally exhibits at least one ethylenic or acetylenic unsaturation,
(iv) an optionally substituted cycloalkyl having from 3 to 12 carbon atoms which optionally exhibits at least one ethylenic or acetylenic unsaturation,
(v) an optionally substituted aryl, an optionally substituted naphthyl, an optionally substituted aryl($C_1$-$C_{30}$ alkyl) or an optionally substituted ($C_1$-$C_{30}$ alkyl)aryl,
it being possible for the radicals (ii) to (v) to be substituted by halogen atoms, organometallic compounds, alcohol, amine, carboxylic, sulfonic, sulfuric, phosphoric, phosphonic or hydroxamic acid or ester, carbamate, thiocarbamate, ether, thiol, epoxide, thioepoxide, isocyanate or isothiocyanate functional groups or it being possible for a carbon of these radicals to be replaced by a nitrogen, sulfur, phosphorus, oxygen, boron or arsenic heteroatom,
(vi) a polysaccharide.

**4.** The dermocosmetic composition of any one of claims 1 to 3, comprising from 2 to 30 mg of calixarene per gram of the composition.

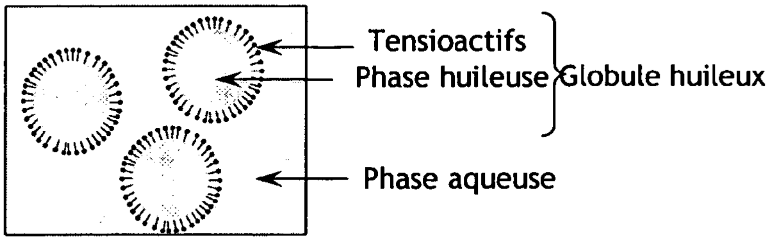


Figure 1

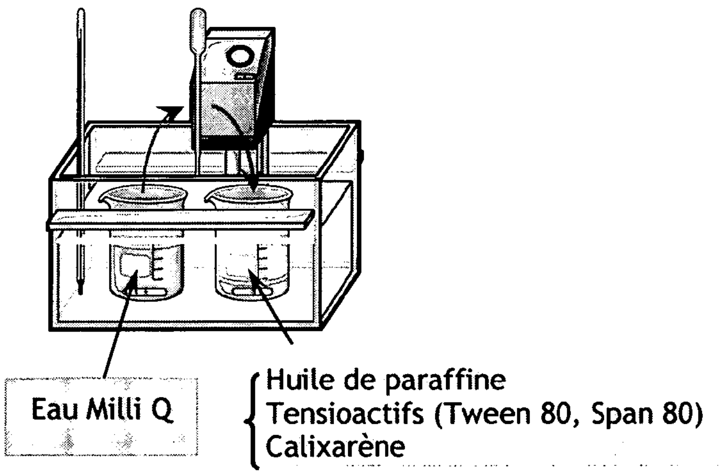


Figure 2

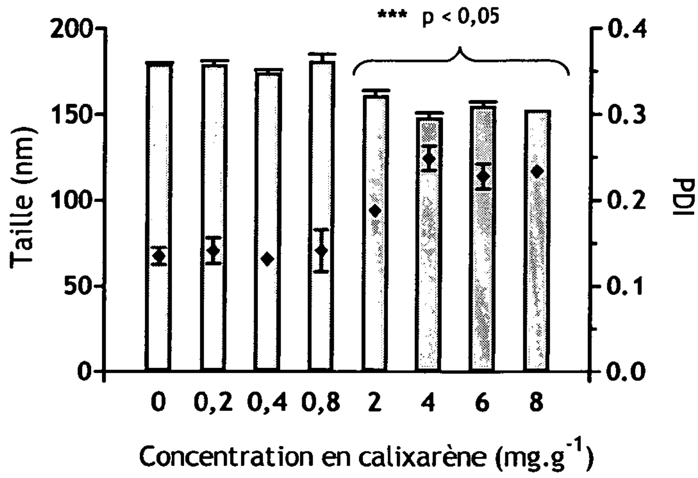


**Figure 3**

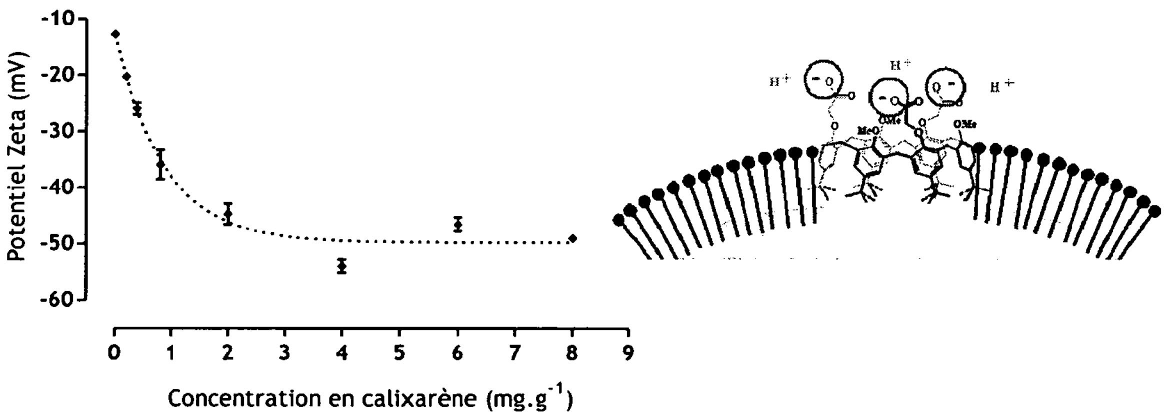


**Figure 4**

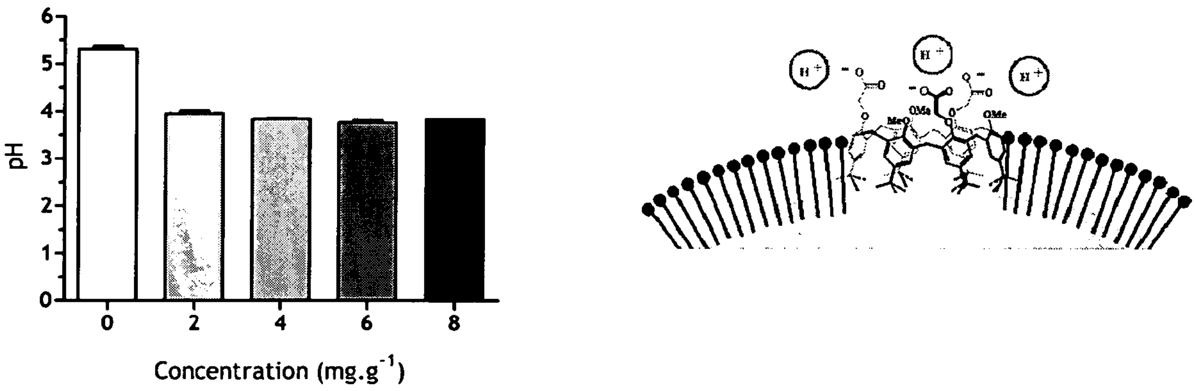


Figure 5

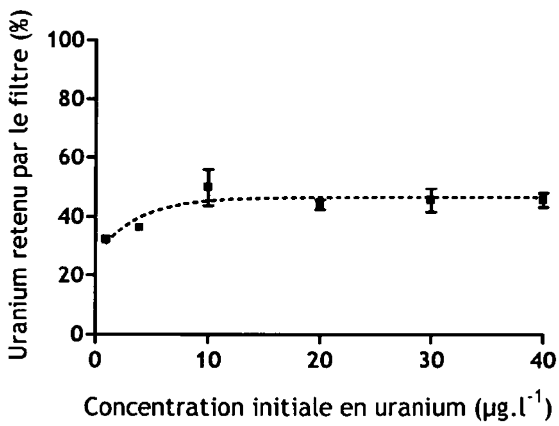


Figure 6

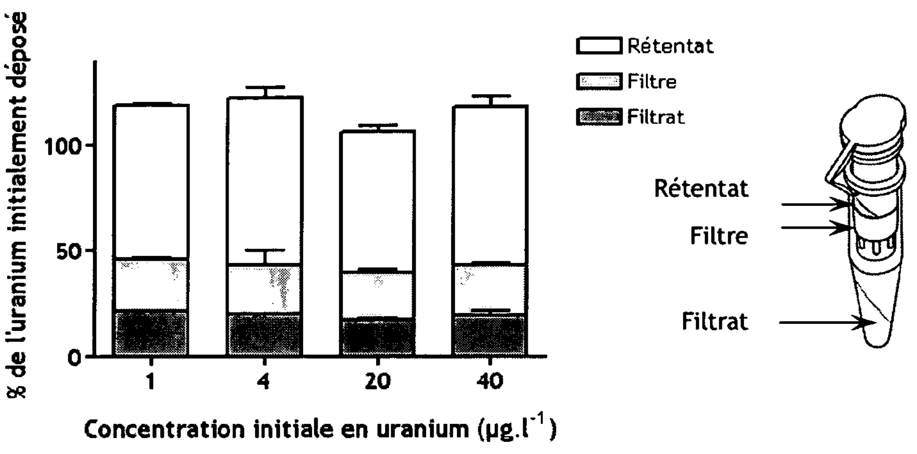


**Figure 7**

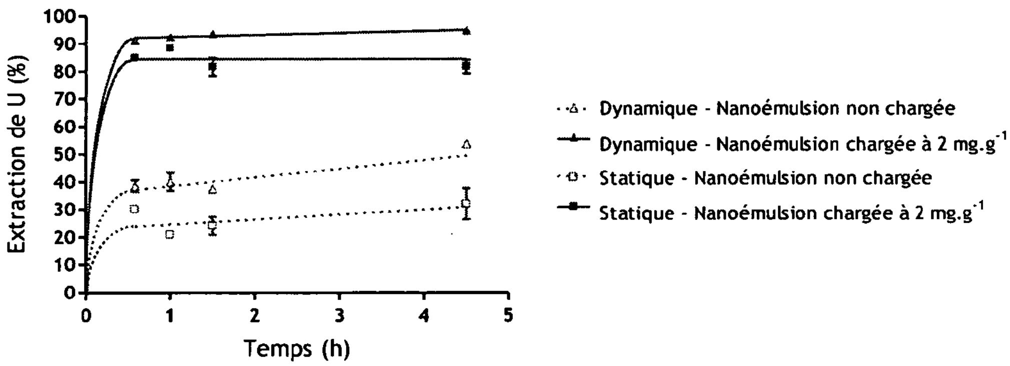


**Figure 8**

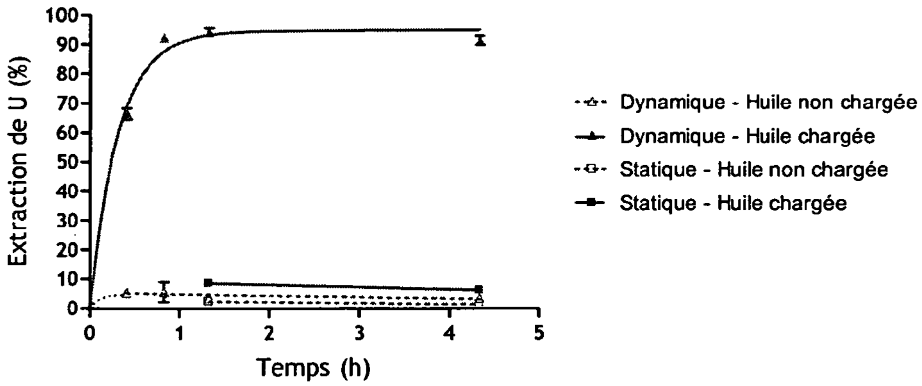
100
90
80
70
60
50
40
30
20
10
0
Extraction de U (%)
0
1
2
3
4
5
Temps (h)
Dynamique - Huile non chargée
Dynamique - Huile chargée
Statique - Huile non chargée
Statique - Huile chargée

# Figure 9

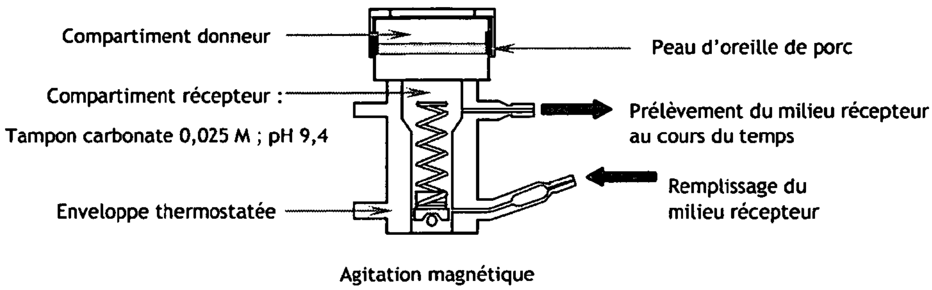
Compartiment donneur
Peau d'oreille de porc
Compartiment récepteur :
Tampon carbonate 0,025 M ; pH 9,4
Prélèvement du milieu récepteur au cours du temps
Remplissage du milieu récepteur
Enveloppe thermostatée
Agitation magnétique

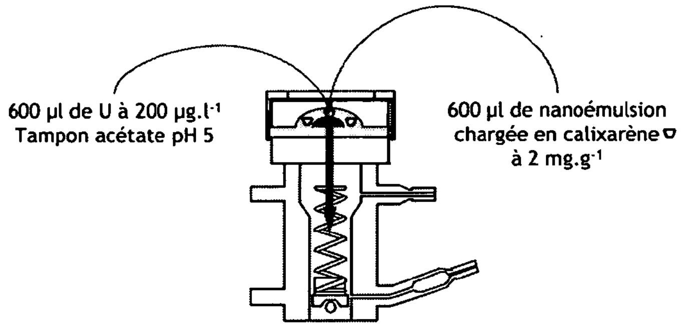
600 µl de U à 200 µg.l-1
Tampon acétate pH 5
600 µl de nanoémulsion chargée en calixarène
à 2 mg.g-1

# Figure 10

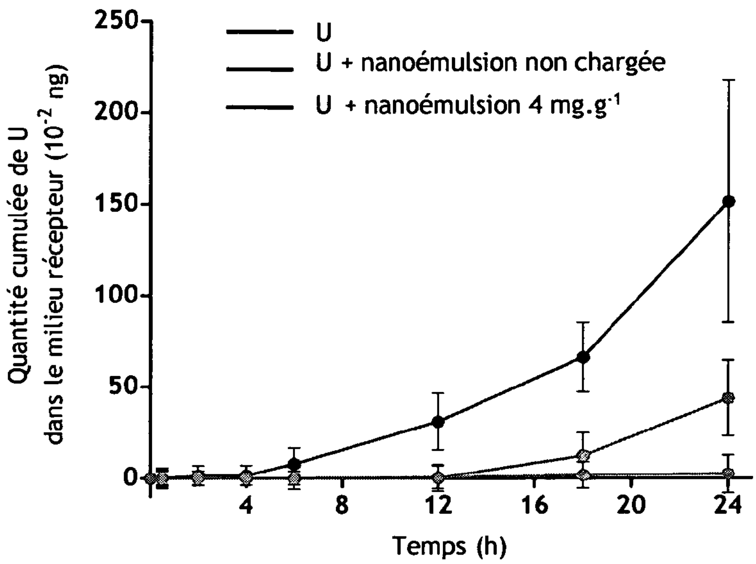
U
U + nanoémulsion non chargée
U + nanoémulsion 4 mg.g-1
Quantité cumulée de U dans le milieu récepteur (10-2 ng)
250
200
150
100
50
0
4
8
12
16
20
24
Temps (h)

**Figure 11**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2006123051 A
- WO 9519974 A
- US 5441983 A
- FR 2782007
- WO 0244121 A
- WO 2004085442 A
- WO 03024583 A


### Littérature non-brevet citée dans la description


- **ARCHIMBAUD M et al.** Radiation Protection Dosimetry. Nuclear Technology Publishing, 01 Janvier 1994, vol. 53
- *Chem. Lett.,* 1993, 829-832
- *J. Am. Chem. Soc.,* vol. 116, 5814-5822
- *Radioprotection,* vol. 32 (5), 659-671
- Uranium et ses composés. **LE GUEN B ; BÉRARD P.** Toxicologie-Pathologie professionnelle. Elsevier, 1998
- **PETITOT F ; MOREELS AM ; PAQUET F.** In vitro évaluation of percutaneous diffusion of uranyl nitrate through intact or excoriated skin of rat and pig. *Can J Physiol Pharmacol,* 2004, vol. 82 (2), 133-9
- **TYMEN H ; GERASIMO P ; HOFFSCHIR D.** Contamination and decontamination of rat and human skin with plutonium and uranium, studied with a Franz's chamber. *Int J Radiat Biol,* 2000, vol. 76 (10), 1417-24
- **TYMEN H ; RATEAU G ; GUILLET K ; RAMOUNET-LE GALL B ; GERASIMO P ; FRITSCH P.** Methods to measure transfer of radionuclides through intact or injured skin -Application to radiotoxicotogy [Méthodes de mesure du transfert cutané des actinides au travers d'un épiderme intact ou lésé, application à la radiotoxicologie. *Canadian Journal of Physiology and Pharmacology,* 2002, vol. 80 (7), 733-741
- **DE REY BM ; LANFRANCHI HE ; CABRINI RL.** Percutaneous absorption of uranium compounds. *Environmental Research,* 1983, vol. 30 (2), 480-491
- **DURBIN PW ; KULLGREN B ; XU J ; RAYMOND KN.** Development of Decorporatiàn Agents for the Actinides. *Radiation Protection Dosimetry,* 1998, vol. 79 (1-4), 433-443
- **FUKUDA S ; LIDA H ; IKEDA M ; YAN X ; XIE Y.** Toxicity of uranium and the removal effects of CBMIDA and EHBP in simulated wounds of rats. *Health Phys,* 2005, vol. 89 (1), 81-8
- **HENGE-NAPOLI MH ; ANSOBORLO E ; CHAZEL V ; HOUPERT P ; PAQUET F ; GOURMELDN P.** Effiacy of ethane-1-hydroxy-1,1-bisphosphonate (EHBP) for the decorporation of uranium after intramuscular contamination in rats. *Int J Radiat Biol,* 1999, vol. 75 (11), 1473-7
- **HOUPERT P ; CHAZEL V ; PAQUET F ; BAILLY T ; BURGADA R.** Henge-Napoli MH Réduction of uranium transfer by local chelation iri simulated wounds in rats. *Hum Exp Toxicol,* 2001, vol. 20 (5), 237-41
- **HOUPERT P ; CHAZEL V ; PAQUET F.** A local approach to reduce industrial uranium wound contamination in rats. *Can J Physiol Pharmacol,* 2004, vol. 82 (2), 73-8
- **YANG Z ; XU K ; WANG L ; GU H ; WEI H ; XANG M ; XU B.** Self-assembly of small molecules affords multifonctional supramolecular hydrogels for topically treating simulated uranium wounds. *Chem Commun,* 2005, vol. 35, 4414-4416
- **SHAN Z ; YANG Y-Z ; TAO Z ; JIAN H ; CHANG-HONG L.** Uranium(VI) extraction by Winsor microemulsion Systems using trialkyl phosphine oxide. *Journal of Radioanalytical and Nuclear Chemistry,* 2005, vol. 265 (3), 419-421
- **GUO J-X ; SUN X ; DU D-I ; WU X ; LI M-X ; PANG H ; SUN S-X ; WANG A-HI.** Uranium(VI) extraction from chloride solution with benzyloctadecyldimethyl ammonium chloride (BODMAC) in a liquid membrane process. *Journal of Radioanalytical and Nuclear Chemistry,* 2008, vol. 275 (2), 365-369
- **SEILLER M ; MARTINI MC.** Formes pharmaceutiques pour application locale. *Lavoisier,* 1996
- **MARTINI M-C.** Introduction à la dermopharrnacie et à la cosmétologie. *Lavoisier,* 2006, 411
- **GUO L ; WARNKEN KW ; SANTSCHI PH.** Rétention behavior of dissolved uranium during ultrafiltration: Implications for colloïdal U in surface waters. *Marine Chemistry,* 2007, vol. 107 (2), 156-166

• **LIU W ; SUN D ; LI C ; LIU Q ; XU J.** Formation and stability of paraffin oil-in-water nano-emulsions prepared by the emulsion inversion point metbod. *Journal of Colloid and Interface Science,* 2006, vol. 303 (2), 557-563